(19) Europäisches Patentamt
European Patent Office
Office européen des brevets

(11) **EP 3 510 922 B1**

(12) **EUROPEAN PATENT SPECIFICATION**

(45) Date of publication and mention
of the grant of the patent:
**15.11.2023 Bulletin 2023/46**

(21) Application number: **17848754.2**

(22) Date of filing: **05.09.2017**

(51) International Patent Classification (IPC):
***A61B 5/00*** *(2006.01)*

(52) Cooperative Patent Classification (CPC):
**A61B 5/6823; A61B 5/24; A61B 5/25; A61B 5/291;
A61B 5/332; A61B 5/6831**

(86) International application number:
**PCT/JP2017/031934**

(87) International publication number:
**WO 2018/047814 (15.03.2018 Gazette 2018/11)**

(54) **BIOSIGNAL DETECTION GARMENT**

**BIOSIGNALDETEKTIONSBEKLEIDUNG**

**SOUS-VÊTEMENT DE DÉTECTION DE BIOSIGNAL**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.09.2016 JP 2016174290**

(43) Date of publication of application:
**17.07.2019 Bulletin 2019/29**

(73) Proprietor: **Nippon Telegraph And Telephone
Corporation
Chiyoda-ku,
Tokyo 100-8116 (JP)**

(72) Inventors:
• **OTSUKA, Azuki**
**Osaka-shi**
**Osaka 530-8222 (JP)**
• **ISHIKAWA, Emiko**
**Osaka-shi**
**Osaka 530-8222 (JP)**
• **MURAKAMI, Yasuharu**
**Osaka-shi**
**Osaka 530-8222 (JP)**
• **ISHIHARA, Takako**
**Musashino-shi**
**Tokyo 180-8585 (JP)**
• **TAKAGAHARA, Kazuhiko**
**Musashino-shi**
**Tokyo 180-8585 (JP)**
• **FUJII, Kouji**
**Musashino-shi**
**Tokyo 180-8585 (JP)**

(74) Representative: **Mewburn Ellis LLP
Aurora Building
Counterslip
Bristol BS1 6BX (GB)**

(56) References cited:
JP-A- 2005 065 937    JP-A- 2009 510 276
JP-A- 2014 226 367    US-A1- 2004 009 731
US-A1- 2005 049 515    US-A1- 2008 287 770
US-A1- 2013 281 795    US-A1- 2014 343 390

EP 3 510 922 B1

# EP 3 510 922 B1

## Description

The present invention relates to a biosignal detecting garment for detecting biosignals including an electrical activity of the heart.

### Background

In recent years, intensive studies have been made toward a technique for attaching a wireless electronic device to the human body as the wireless electronic device becomes smaller. As an individual health management method, various types of biosignal detecting garments (also referred to as wearable electrodes) capable of detecting biosignals such as an electrical activity of the heart, a heart rate, and an electrical activity of the muscle for a long period of time have been studied.

Specifically, there are proposed a garment, such as a shirt and underpants, which can receive biosignals using an electroconductive fiber structure installed in a stretchable fabric portion of the garment so that the structure is brought into local close contact with the living body (for example, see Patent Literature 1), a garment which includes a sensor and an electronic device disposed thereon, and a stretchable electrical wire arranged therebetween (for example, see Patent Literature 2), a washable heart rate monitoring garment to which an electrode, a transmitter, and a power supply are attached (for example, see Patent Literature 3), a bodysuit which includes a belt laterally stretchable relative to a rising direction of a wearer and an electrode using an elastomer material as a base material for measuring the electrical activity of the heart or the skin resistance, the electrode being disposed in the belt (for example, see Patent Literature 4), a garment which includes electrodes for bipolar limb lead provided on inner surfaces of both shoulder portions and a belt-shaped woven or knitted fabric for fastening the garment to a chest portion, a torso portion, and the like (for example, see Patent Literature 5), an electrode-attached brassiere in which an electrode portion prepared by laminating electrode films on right and left portions of a base film extended in the lateral direction and further coating a central portion between the electrodes with an insulating layer is disposed on an inner surface of an under belt to bring the electrode films into contact with the body and thereby acquire information on an electrical signal generated by the heart (for example, see Patent Literature 6), a textile based electrode including a knitted electrode, wherein the textile based electrode can be included in a wearable textile, such as a waist band, a vest, a bra (for example, see Patent Literature 7), and a circularly knit garment having an inner surface with a discrete electrically conductive region to contact the wearer's skin (for example, see Patent Literature 8).

### Citation List

Patent Literature 1: JP 2015-100673 A; Patent Literature 2: JP 2012-188799 A; Patent Literature 3: U.S. Patent No. 8224418; Patent Literature 4: Japanese Translation of PCT Patent Application Publication No. 2008-503287; Patent Literature 5: JP 6-70896 A; Patent Literature 6: Japanese Design Registration No. 1552253; Patent Literature 7: US 2014/0343390 A; and Patent Literature 8: US 2014/0009731 A.

### Summary

### Technical Problem

The biosignal detecting garment is required to stably detect biosignals for a long period of time and provide a wearing feeling with no discomfort. However, in the a garment, such as a shirt and underpants, which can receive the biosignals using the electroconductive fiber structure installed in the stretchable fabric portion of the garment so that the structure is brought into local close contact with the living body (Patent Literature 1) and the garment which includes the sensor and the electronic device disposed thereon, and the stretchable electrical wire arranged therebetween (Patent Literature 2), the garments are formed in the shirt or underpants shape. Thus, although the clothing fabric and the electrical wire are made stretchable, the electrode may be detached from the body due to the height of the bust or chest during the detection of the biosignals, making it difficult to stably detect the biosignals. Further, another problem is that wearing the brassiere makes it difficult to detect the biosignals as it overlaps with the electrode installed in the garment described above.

Further, in the bodysuit which includes the belt laterally stretchable relative to the rising direction of the wearer and the disk-shaped electrode formed by a mass of an elastomer with electroconductive particles embedded therein for measuring the electrical activity of the heart or the skin resistance, the electrode being disposed in the belt (Patent Literature 4), the garment which includes the electrodes formed by non-fiber structures for bipolar limb lead on the inner surfaces of both shoulder portions and the belt-shaped woven or knitted fabric for fastening the garment to a chest portion, a torso portion, and the like (Patent Literature 5), and the electrode-attached brassiere in which the electrode portion prepared by laminating the electrode films on the right and left portions of the base film extended in the lateral direction and further coating the central portion between the electrodes with the insulating layer is disposed on the inner surface of the under belt to bring the electrode films into contact with the body and thereby acquire the information on the electrical signal generated by the heart (Patent Literature 6), the electrodes are not configured from the fiber structures. This makes it difficult to wash the garment and wear the garment repeatedly (for a long period of time) or causes discomfort to the user who continues to wear the garment due to poor texture, thus creating issues.

In view of the aforementioned conventional techniques, the present invention provides a biosignal detecting garment capable of continuously and stably detecting a biosignal for a long period of time without causing discomfort.

Solution to Problem

In order to solve the above-described problems and achieve the object, there is provided according to the present invention a biosignal detecting garment comprising:

a crop top type or brassiere type garment body;an under belt that have a fastening tool capable of adjusting a chest circumference size and that is disposed in a lower portion of the garment body; at least two or more electrodes formed of an electroconductive fiber;
a connector for installing a measurement device configured to detect a biosignal; and
a wiring portion configured to electrically connect the electrodes and the connector, wherein
the electrodes, the connector, and the wiring portion are installed in the under belt,
the garment body has a length from an apex of a shoulder portion of the garment body to an upper portion of the under belt of 20.0 cm or more and 35.0 cm or less,
the under belt is formed in a bag shape with two pieces stacked, andan opening portion with a range of 1.0 cm or more and 10.0 cm or less is provided at a center of a front body piece.

Advantageous Effects of Invention

In the biosignal detecting garment according to the present invention, the electrodes, the connector, and the wiring portion are installed in the under belt that is disposed in a lower portion of the crop top type or brassiere type garment body, whereby the biosignal detecting garment can continuously and stably detect biosignals for a long period of time without causing discomfort during wearing.

Brief Description of Drawings

FIG. 1 includes schematic views illustrating a crop top type biosignal detecting garment according to a first embodiment of the present invention.

FIG. 2 includes schematic views illustrating a brassiere type biosignal detecting garment according to a second embodiment of the present invention.
FIG. 3 is a cross-sectional view of an under belt of the biosignal detecting garment of FIG. 1 taken along line X-X' .
FIG. 4 is a schematic view illustrating the crop top type biosignal detecting garment according to the first embodiment of the present invention worn on top of a brassiere.
FIG. 5 includes schematic views each illustrating a measurement site with a length from a neck point or an apex of a strap to an upper portion of the under belt in the crop top type or brassiere type biosignal detecting garment according to the first or second embodiment of the present invention.
FIG. 6 is a schematic view illustrating a chest belt type (brassiere type) biosignal detecting garment according to a third embodiment of the present invention.

Description of Embodiments

Hereinafter, a biosignal detecting garment according to the present invention will be described in detail on the basis of the drawings. It should be noted that the present invention is not limited by the embodiments.

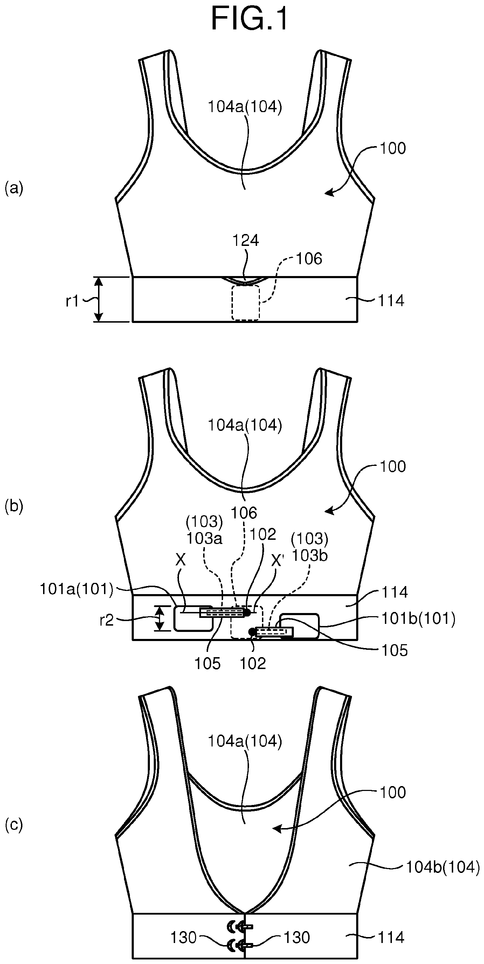

FIG. 1 includes schematic views illustrating a crop top type biosignal detecting garment 100 according to a first embodiment of the present invention. The crop top type biosignal detecting garment 100 refers to a garment that includes a garment body 104 including a front body piece 104a and a back body piece 104b, and an under belt 114 as parts. Further, a member such as a strap may be used to connect the front body piece 104a and the back body piece 104b.

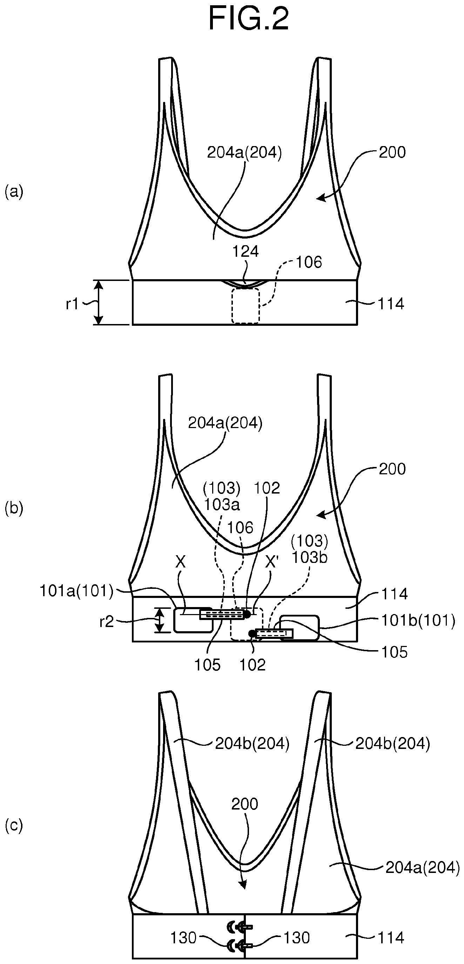

Further, FIG. 2 includes schematic views illustrating a brassiere type biosignal detecting garment 200 according to a second embodiment of the present invention. The brassiere type biosignal detecting garment 200 refers to a garment that includes a garment body 204 including a front body piece 204a and straps 204b, and the under belt 114 as parts.

FIG. 1(a) and FIG. 2(a) are front views (front body piece sides), FIG. 1(b) and FIG. 2(b) are reverse side views of the front body pieces (sides in contact with the skin), and FIG. 1(c) and FIG. 2(c) are back side views (back body piece sides).

Note that, hereinafter, the crop top type biosignal detecting garment 100 and the brassiere type biosignal detecting garment 200 may be referred to as the biosignal detecting garment 100 and the biosignal detecting garment 200, respectively.

As shown in FIG. 1, the crop top type biosignal detecting garment 100 of the present invention includes the garment body 104, the under belt 114 disposed in the lower portion of the garment body 104, at least two electrodes 101a and 101b (101) formed of an electroconductive fiber, a connector 102 for mounting a measurement device 106 that detects biosignals, and wiring portions 103a and 103b (103) for electrically connecting the electrodes 101a and 101b and the connector 102. The electrodes 101a and 101b, the connector 102, and the wiring portions 103a and 103b are installed in the under belt 114.

As shown in FIG. 2, the brassiere type biosignal detecting garment 200 of the present invention includes the garment body 204, the under belt 114 disposed in the lower portion of the garment body 204, at least two electrodes 101a and 101b (101) made of electroconductive fibers, a connector 102 for mounting a measurement device 106 that detects a biosignal, and wiring portions 103a and 103b (103) for electrically connecting the electrodes 101a and 101b and the connector 102. The electrodes 101a and 101b, the connector 102, and the wiring portion 103 are installed in the under belt 114.

In this specification, the installation is not limited to a particular method as long as the under belt 114 and the electrodes 101a and 101b, the under belt 114 and the connector 102, and the under belt 114 and the wiring portions 103a and 103b are in contact with each other and fixed to each other. As an example of the method, bonding using a hot melt adhesive, sewing using threads, or fixing via a metal snap fastener may be used.

In the crop top type biosignal detecting garment 100 and the brassiere type biosignal detecting garment 200 of the present invention, the electrodes 101a and 101b that detects the biosignals from the body are formed of the electroconductive fiber. The electroconductive fiber is preferably a fiber structure impregnated with an electroconductive polymer. It is more preferable that the fiber structure includes a multifilament structure, and the electroconductive resin is supported on the surfaces of the filaments constituting the fiber structure and the gaps between the filaments. In conventional film type electrodes commonly used as electrodes for electrocardiograms, it has been inevitable to apply an acrylic gel to the surfaces of the electrodes in order to improve adhesion to the body and obtain electrical signals, and thus, there is a problem that skin damage is likely to occur. On the other hand, the electrodes 101a and 101b formed of the fiber structure according to the present invention are less irritating when being in contact with the skin and thus have high safety.

The electroconductive polymer used in the electrodes 101a and 101b according to the present invention is not particularly limited as long as it is a resin having electroconductivity. There are electroconductive resin compositions containing an electroconductive polymer such as PEDOT/PSS, carbon black, CNT (Carbon Nanotube), metallic fine particles, and the like. However, in the case of using a stretchable resin such as an elastomer resin, the conductivity is changed due to the degree of stretching and shrinking, and it becomes difficult to stably detect a signal. Thus, such a stretchable resin is not preferable. As the electroconductive polymer used for the electrodes 101a and 101b, a PEDOT/PSS in which a PEDOT of a thiophenebased electroconductive polymer, which itself is an electroconductive polymer having electroconductivity, is doped with polystyrene sulfonic acid (poly 4-styrene sulfonate; PSS) is more preferable from the viewpoints of safety and processability.

Examples of the form of the fiber structure used for the electrodes 101a and 101b may include textiles such as knitted fabrics, woven fabrics and nonwoven fabrics, and cords. Preferably, knitted or woven fabrics are used. In addition, if the quantity of the electroconductive resin to be impregnated into the fiber structure is insufficient, washing durability in repeated use cannot be obtained, and therefore, the areal weight of the fiber structure is preferably 50 g/m$^2$ or more and 300 g/m$^2$ or less. If the areal weight is less than 50 g/m$^2$, the amount of the processing liquid containing the electroconductive resin to be held is reduced when the processing liquid is applied to the fiber structure, so that the amount of the electroconductive resin to be impregnated is reduced and the washing durability cannot be obtained. If the areal weight is larger than 300 g/m$^2$, the substantial areal weight becomes large, causing uncomfortable wearing feeling. More preferably, the areal weight is 60 g/m$^2$ or more and 250 g/m$^2$ or less.

Examples of the fiber material used in the fiber structure of the present invention may include synthetic fibers such as fibers made of polyethylene terephthalate, polypropylene terephthalate, polybutylene terephthalate, and the like, aromatic polyester-based fibers obtained by copolymerizing a third component with the aforementioned components, aliphatic polyester-based fibers typified by L-lactic acid as a main component, polyamide-based fibers such as nylon 6 and nylon 66, acrylic-based fibers containing polyacrylonitrile as a main component, polyolefin-based fibers such as polyethylene and polypropylene, and polyvinyl chloride-based fibers. Fibers containing additives such as titanium oxide, or fibers modified with a polymer for imparting a function such as improved hygroscopicity may also be used.

From the viewpoint of supporting electroconductive resins on the surfaces of filaments and in the gaps between filaments, the fiber structures according to the present embodiment preferably include a multifilament having a fineness of the filament of 0.2 dtex or less. The mixing ratio of the multifilament including the filament of 0.2 dtex or less in the fiber structure is not particularly limited as long as it does not affect the performance, but higher mixing ratio is preferable from the viewpoints of electroconductivity and durability. The mixing ratio is more preferably 50% or more and 100% or less. Further, the larger the number of filaments is, the more the gaps formed by the plurality of filaments, that is, sites on which the electroconductive resin is supported, are subdivided, so that the electroconductive resin is supported on the fiber structure in a higher degree. In addition, even when the site is subdivided by the smaller fiber diameter, the continuity of the electroconductive resin is maintained, so that excellent high conductivity and washing durability can be obtained.

It is preferable to use microfibers having a fiber diameter of 5 $\mu$m or less used for artificial leather, materials for outerwear, or the like, and more preferable to use nanofibers having a fiber diameter of 10 nm or more and 1000 nm or less.

As the nanofiber, a fiber structure containing nanofibers produced by known methods such as a nanofiber staple yarn aggregate produced from "NANOALLOY (registered trademark)" fibers, an aggregate of monofilament yarn produced by an electrospinning method, or the like, can be suitably used, but a fiber structure containing multifilament yarn of nanofibers is more preferable. The multifilament yarn of nanofibers can be produced by a known composite spinning method or the like. As an example, nanofiber multifilament yarn having a small variation in fiber diameter obtained by removing sea components from composite fibers using a composite spinneret exemplified in Japanese Patent Application Laid-Open No. 2013-185283 can be effectively used, but the present invention is not limited thereto.

Further, it is preferable that the electrodes 101a and 101b used in the present invention are formed by layering a resin layer on one surface of the fiber structure containing the electroconductive material. In consideration of adaptation to the biological electrode, it is preferable that the resin layer is layered on the opposite surface side to the surface of the fiber structure used for the electrodes 101a and 101b, which is in contact with the skin. When the electroconductive resin is applied to the fiber structure, since the amount of the processing liquid supported by the fiber structure is increased by layering the resin layer on one surface of the fiber structure, the impregnation amount of the electroconductive resin is increased, so that excellent washing durability and high conductivity can be obtained.

The type and shape of a polymer constituting the resin layer are not particularly limited as long as a humidity control can be performed. However, a moisture permeable layer is preferable. If a movement of the moisture is completely interrupted, a stuffy feeling becomes stronger, thereby resulting in an uncomfortable feeling during wearing. Interruption of the moisture movement also causes a skin rash or the like. Examples of the moisture permeable layer may include, but not limited to, layers formed by laminating known membranes such as a polytetrafluoroethylene (PTFE) porous membrane, a nonporous membrane formed of a hydrophilic elastomer such as a hydrophilic polyester resin and polyurethane resin, and a polyurethane resin microporous membrane, known films, known laminated products, known resins, and the like using a coating or laminating method. The moisture permeable layer is preferably formed by laminating and adhering the stretchable polyurethane resin microporous membrane using the laminating method from the viewpoint of followability to the fiber structure serving as the base material.

The size and shape of the electrodes 101a and 101b are not particularly specified as long as biosignals can be detected, and it is preferable that each of the lengths in the longitudinal and lateral directions is 2.0 cm or more and 20.0 cm or less. If each of the lengths in the longitudinal and lateral directions of the electrodes 101a and 101b is smaller than 2.0 cm, the area of the electrode is too small, so that the contact resistance increases due to the decreased contact area between the electrode and the human body, and the electrode also tends to be displaced when the clothing fabric moves during exercise or the like, so that noise can be easily picked up. If it is larger than 20.0 cm, such a size is not necessary for actual signal detection, and the area of the electrode is too large. Thus, the distance between adjacent electrodes is small, so that it is likely to cause troubles such as a short circuit. Each of the lengths thereof in the longitudinal and lateral directions is more preferably 2.0 cm or more and 18.0 cm or less.

The measurement device 106 used in the crop top type biosignal detecting garment 100 and the brassiere type biosignal detecting garment 200 of the present invention is preferably attached and connected to, and detached from, the garment bodies 104 and 204 via the connector 102. Further, detaching the measurement device 106 from the garment bodies 104 and 204 enables washing. The connector 102 is not particularly limited, and a socket or the like commonly used for connecting cords may be used. It is more preferable to use a plurality of metal snap fasteners that can simultaneously fix the measurement device 106 to the garment bodies 104 and 204.

The measurement device 106 preferably has a function of transferring data through communication with a mobile terminal or a personal computer. This function enables the data to be easily acquired, stored, and analyzed in a personal computer, for example. In addition, it is particularly preferable that the measurement device 106 communicates with a mobile terminal or a personal computer through wireless communication. Wireless communication eliminates the need to bind users for communication.

In the crop top type biosignal detecting garment 100 and the brassiere type biosignal detecting garment 200 of the present invention, the wiring portions 103a and 103b that transmit the biosignals obtained by the electrodes 101a and 101b to the measurement device 106 are required. The wiring portions 103a and 103b are preferably formed by a method of printing an electroconductive resin on the under belt 114 of the garment bodies 104 and 204, a method of laminating a film of an electroconductive resin, and further a method of forming them from an electroconductive fiber or a metal wire.

When the wiring portions 103a and 103b are formed of the electroconductive fiber, yarn in which polyester or nylon fibers are covered with metal fibers including silver, aluminum or stainless steel, an electroconductive fiber in which carbon black is combined and disposed on a portion of a core or sheath of polyester or nylon in the length direction of the fiber, or metal-coated yarn in which polyester or nylon fibers are coated with metals including silver, aluminum or stainless steel may be used as the electroconductive fibers. From the viewpoint of durability and versatility, it is particularly preferable to use yarn in which polyester or nylon fibers are covered with metal fibers including silver, aluminum or stainless steel.

It is preferable that the wiring portions 103a and 103b formed by printing electroconductive fibers, electroconductive resins, or the like are disposed on the surface of the under belt 114 (the surface not in contact with the skin), or that the wiring portions 103a and 103b disposed on the back surface of the under belt 114 (the surface in contact with the skin) are covered with an electrically insulating member 105. Covering the wiring portions 103a and 103b disposed on the front surface or the back surface with the electrically insulating member 105 prevents the wiring portions 103a and 103b from coming into direct contact with the skin, and noise acquired by the wiring portions 103a and 103b from being mixed into the biosignals detected by the electrodes 101a and 101b, whereby the biosignals can be accurately measured. Further, from the viewpoint of design, if the wiring portions 103a and 103b are provided on the surface of the under belt 114, the wiring portions 103a and 103b or the electrically insulating member 105 are exposed. In order to prevent the wiring portions 103a and 103b and the electrically insulating member 105 from being exposed, it is preferable to cover the wiring portions 103a and 103b disposed on the back surface of the under belt 114 with the electrically insulating member 105.

As a method of attaching the electroconductive fibers used for the wiring portions 103a and 103b to the under belt 114 of the garment bodies 104 and 204, it is preferable that the wiring portions 103a and 103b formed of an electroconductive tape obtained by weaving the electroconductive fibers in a belt shape are sandwiched between the electrically insulating member 105 having the electrically insulating property to which a hot melt adhesive has been applied on one side and the fabric of the under belt 114, and are attached by thermal bonding. In addition, in sewing using a sewing machine, it is possible to sew with the back surface of the clothing fabric facing upward using an electroconductive fiber as a bobbin thread and a normal sewing machine thread as a needle thread. As a result, the electroconductive fibers are mainly exposed on the surface on the side where the under belt 114 is not in close contact with the skin. Further, as a stitching method of the electroconductive fibers, it is preferable to stitch the electroconductive fibers to the under belt 114 by catch stitching. Sewing the electroconductive fibers by catch stitching enables the sewing thread portions to move even during stretching and shrinking of the clothing fabric, and to follows the clothing fabric, so that the stretchability is not hindered.

In the crop top type biosignal detecting garment 100 and the brassiere type biosignal detecting garment 200 of the present invention, since polarization can be suppressed by connecting the electrodes 101a and 101b and the wiring portions 103a and 103b formed of these electroconductive fiber structures, this is not only preferable for detecting weak biosignals but can prevent corrosion (electrolytic corrosion), so that continuous use for a long period is possible. The method of connecting the electrodes 101a and 101b and the wiring portions 103a and 103b is not particularly limited, and examples thereof may include a method in which after the wiring portions 103a and 103b are superimposed on the electrodes 101a and 101b disposed on the under belt 114, the contact portions are sewn using a sewing machine, a method in which the electrodes 101a and 101b are superimposed and printed when an electroconductive resin is printed to form the wiring portions 103, a method in which, when the wiring portions 103a and 103b are formed using an electroconductive resin film, a hot melt adhesive is applied onto one surface of the electroconductive resin film, and the film is thermocompression bonded onto the electrodes 101a and 101b to form the wiring portions 103, and a method in which the wiring portions 103 and the electrodes 101a and 101b are connected via metal snap fasteners.

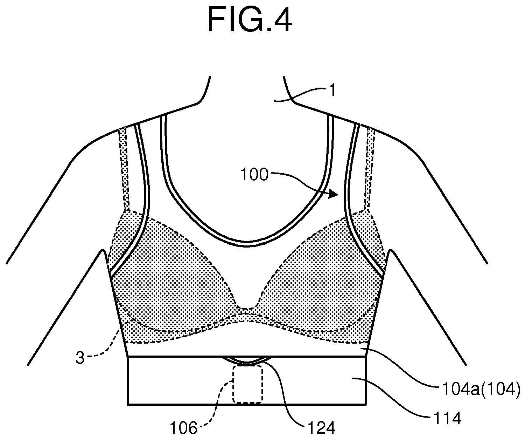

The wiring portions 103a and 103b of the present invention are required to transmit the biosignals obtained from the electrodes 101a and 101b to the measurement device 106 with high sensitivity. The reason why the wiring portions 103a and 103b become unable to transmit the biosignals with high sensitivity is considered due to the intrusion of noise. The noise is caused by detection of the electrical activity of the heart, the electrical activity of the muscle, or the like at a site where the electrodes are not installed due to the contact between a body 1 (see FIG. 4) and the wiring portions 103a and 103b or electric leakage of the biosignal due to the contact with the water content such as rain and sweat. In order to overcome the noise caused by the above factors, the wiring portions 103a and 103b are preferably covered with waterproof electrically insulating members 105. In particular, it is more preferable that: the electrically insulating members 105 serving as waterproof electrically insulating films are applied with a hot-melt adhesive and bonded on the back surface of the under belt 114 by thermocompression using an iron or a press machine; the wiring portions 103a and 103b are placed on the electrically insulating members 105 thus bonded; and the waterproof electrically insulating members 105 imparted with the hot-melt adhesive are further bonded on the wiring portions 103a and 103b by thermocompression using the iron or the press machine so as to interpose the wiring portions 103a and 103b between the electrically insulating members 105, thereby coating the wiring portions 103a and 103b with the waterproof electrically insulating films on the skin side and the clothing fabric side.

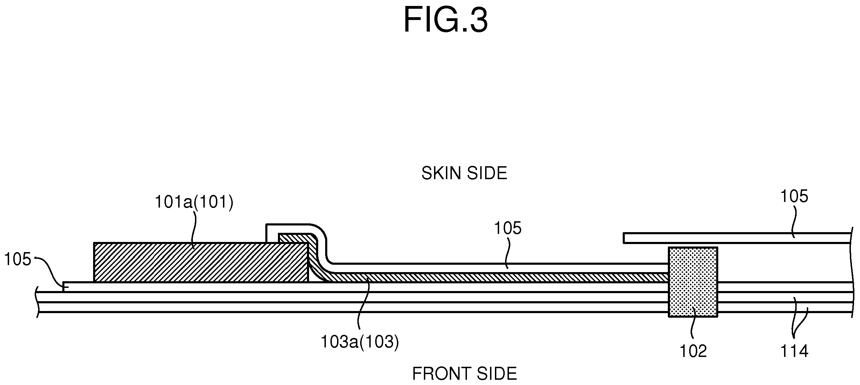

FIG. 3 is a cross-sectional view of the under belt 114 of the biosignal detecting garment 100 of FIG. 1 taken along line X-X' (the same applies to the cross-sectional view of the biosignal detecting garment 200 of FIG. 2 taken along line X-X'). The wiring portions 103a and 103b shown in FIG. 3 are produced by weaving electroconductive fibers in a belt shape. A waterproof electrically insulating member 105 to which a hot-melt adhesive has been applied is thermocompression-bonded by an iron or a press to the back surface of the under belt 114, and then the produced wiring portions 103a and 103b are placed thereon. Further, a waterproof electrically insulating member 105 to which a hot-melt adhesive has been applied is thermocompression-bonded by an iron or a press to sandwich the wiring portions 103a and 103b therebetween, so that the wiring portions 103a and 103b can be formed by covering them with waterproof electrical insulation on the skin side and the clothing fabric side.

In the crop top type biosignal detecting garment 100 and the brassiere type biosignal detecting garment 200, the electrodes 101a and 101b are disposed on the back surface of the under belt 114 (the surface in contact with the skin) on the portions in contact with the vicinity of the left and right under busts at the time of wearing, respectively.

Since the two electrodes 101a and 101b are disposed in the vicinity of the left and right under busts on the back surface of the under belt 114, the electrodes 101a and 101b can be stably kept in contact with the body 1 without being affected by the height of the bust or chest, thereby enabling continuous measurement of biosignals for a long period of time. In addition, the measurement device 106 and the electrodes 101a and 101b are electrically connected to each other by the wiring portions 103a and 103b directly disposed on the under belt 114. Since the wiring portions 103a and 103b are integrated with the under belt 114, the wearer is not uncomfortable, and noise at the time of measurement of the biosignals due to the movement of the wiring portions 103a and 103b can be prevented.

The crop top type biosignal detecting garment 100 and the brassiere type biosignal detecting garment 200 may be worn inside the underwear or worn outside and on top of the underwear. In particular, when a T-shirt or an innerwear built with cups is worn, the biosignal detecting garment is preferably worn inside the T-shirt or the innerwear built with cups, and when the brassiere is worn, the biosignal detecting garment is preferably worn outside the brassiere. When the biosignal detecting garment is worn on top of the brassiere, the under belt 114 is preferably not overlapped with a brassiere 3 and located on the abdomen side as shown in FIG. 4 in order to stably detect the biosignals. FIG. 4 is a schematic view illustrating the crop top type biosignal detecting garment 100 according to the first embodiment of the present invention worn on top of the brassiere 3.

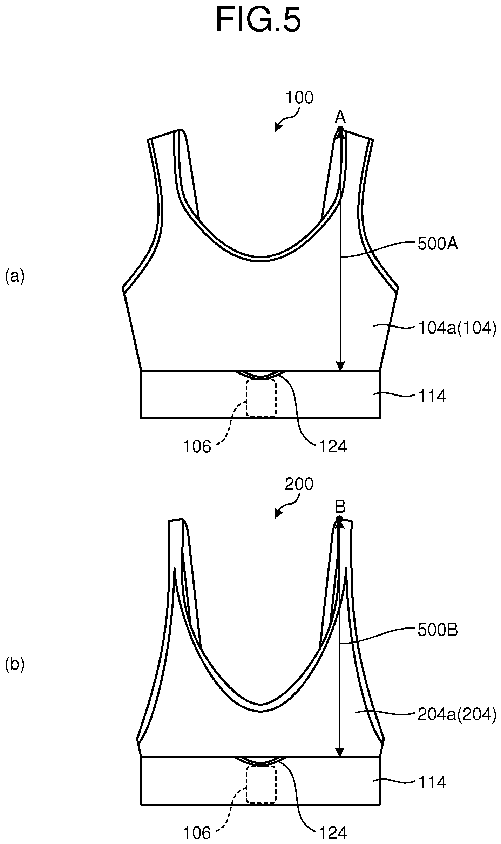

In order to stably detect the biosignals by the electrodes 101a and 101b, as shown in FIG. 5, a length from a neck point A of the shoulder portion or an apex B of the strap to the upper portion of the under belt 114 in the biosignal detecting garment 100 or 200 is according to the present invention 20.0 cm or more and 35.0 cm or less. FIG. 5(a) is a schematic view illustrating a measurement site with a length 500A from the neck point A to the upper portion of the under belt 114 in the crop top type biosignal detecting garment 100 according to the first embodiment of the present invention, while FIG. 5(b) is a schematic view illustrating a measurement site with a length 500B from the apex B of the strap to the upper portion of the under belt 114 in the brassiere type biosignal detecting garment 200 according to the second embodiment of the present invention. If the length from the neck point A of the shoulder portion or the apex B of the strap to the upper portion of the under belt in the biosignal detecting garment 100 or 200 is less than 20.0 cm, the position of the under belt 114 becomes closer to the bust or the chest. As a result, the under belt 114 is susceptible to the height of the bust or the chest and the electrodes are detached from the body, raising concern that it becomes difficult to stably detect the biosignals. Further, as shown in FIG. 4, when the biosignal detecting garment 100 or the like is worn outside the brassiere 3, the under belt 114 is overlapped on top of the brassiere 3 and the electrodes 101a and 101b adhered to the under belt 114 are not brought into direct contact with the skin, making it difficult to detect the biosignals. Further, if the length from the neck point A of the shoulder portion or the apex B of the strap to the upper portion of the under belt 114 is more than 35.0 cm, the under belt 114 is likely to be positioned to the abdomen portion when the biosignal detecting garment 100 or 200 is worn. This raises concern that the noise is generated by detecting the electrical activity of the abdominal muscle during the detection of the electrical activity of the heart. Note that the apex B of the strap refers to a shoulder edge portion of the strap that is stretched without applying any tension in a state that a fastening tool 130 of the under belt 114 is engaged and the lower side of the under belt 114 is aligned.

In the biosignal detecting garments 100 and 200, the width r1 of the under belt 114 is affected by the length r2 in the longitudinal direction of the electrodes 101a and 101b, and in order to adhere the electrodes 101a and 101b to the under belt 114, the width r1 of the under belt 114 must be larger than the length r2 in the longitudinal direction of the electrodes 101a and 101b. Specifically, the width r1 of the under belt 114 is preferably 6.0 cm or more.

In the biosignal detecting garments 100 and 200 of the present invention, it is preferable that the under belt 114 is provided with the fastening tool 130 which can be adjusted in size. In order to stably detect the biosignals as the biosignal detecting garments 100 and 200, it is necessary that the electrodes 101a and 101b are in close contact with the skin when the garments are worn. There are individual differences in the body size of the wearer, and thus, it is preferable to provide two or more fastening tools 130 in order to match the body size of each individual. Dimensionally adjustable fastening tools in this case refer to dimensionally adjustable members such as hooks, hook-and-loop fasteners, adjustment tubes, and the like.

The crop top type biosignal detecting garment 100 and the brassiere type biosignal detecting garment 200 of the present invention are formed in a bag shape in which two under belts 114 are stacked and upper and lower ends are stitched together. According to the present invention, a portion of the upper end of the front-side under belt 114 is not stitched to the skin-side under belt 114 and the garment body 104 and is provided with an opening portion 124. The electrodes 101a and 101b attached to the under belt 114 are configured to read the biosignals and transmit the biosignals to the measurement device 106 attached via the connector 102. If the opening portion 124 is not provided, which is an option not covered by the present invention,

the measurement device 106 is exposed on the surface of the biosignal detecting garments 100 and 200, which is not preferable from the viewpoint of design. Further in this case, the measurement device 106 may be accidentally detached from the connector 102 due to vibration during operation. Providing the opening portion 124 enables the formation of a pocket portion in which the measurement device 106 can be accommodated, so that the measurement device 106 is not exposed on the surface thereof. Further, the measurement device 106 is held down by the pocket formed of the two under belts 114, and the detachment of the measurement device 106 due to the vibration can be suppressed. According to the present invention the width of the opening portion 124 is 1.0 cm or more and 10.0 cm or less so that the measurement device 106 can be easily inserted thereinto and removed therefrom.

The positions of the connector 102, the measurement device 106, and the opening portion 124 are not limited to particular placement locations, and are provided in the middle of the placement location of the electrodes 101a and 101b. Not covered by the present invention is a placement location on either the left or right of the chest or shoulder portion of the garment bodies 104 and 204. By disposing the measurement device 106 or the like on the chest or shoulder portion, it is possible to obtain the effects that noise due to the movement of the user is hardly mixed, that the influence on the daily life activities of the user is small, and that the user can easily attach and detach the measurement device 106. However, if they are disposed at positions distant from the electrodes 101a and 101b, the wiring becomes longer, and the stretchability is impaired by the waterproof electrically insulating member 105 to which the hot melt adhesive used for the wiring portions 103a and 103b has been applied. Thus, it is preferable to dispose them at the middle of the electrodes 101a and 101b where the wiring portions 103a and 103b are shortest.

In the crop top type biosignal detecting garment 100 and the brassiere type biosignal detecting garment 200 of the present invention, it is necessary to bring the electrodes 101a and 101b disposed on the under belt 114 into close contact with the body 1 in order to obtain signals with little noise. The electrodes 101a and 101b are preferably brought into close contact with the body 1 at a compression of at least 0.1 kPa or more and 2.0 kPa or less. If it is larger than 2.0 kPa, although the signal is taken well, the compression becomes large and the wearing feeling becomes worse. If it is 0.1 kPa or less, the electrode is separated from the skin during the operation, and a good signal cannot be obtained. It is more preferably 0.1 kPa or more and 1.0 kPa or less.

In order to achieve the compression described above and wearing comfortableness, it is preferable that the garment bodies 104 and 204 and the under belt 114 are formed by a woven or knitted fabric and an elongation rate of the woven or knitted fabric either in any one of a longitudinal direction and a lateral direction is 30.0% or more and 100.00% or less. The compression can be adjusted by the stretchability and sewing size of the clothing fabric. However, if the elongation rate is less than 30.0%, even when the compression is confined within the range described above by increasing the size of the clothing fabric, the clothing fabric fails to follow the stretching during the body movement and thereby interferes with the body movement. Further, if the elongation rate is more than 100.0%, even when the compression is confined within the range described above by reducing the size of the clothing fabric, the clothing fabric does not have enough strength to follow the stretching during the body movement, which may cause a breakage of the clothing fabric.

In order to realize the above-mentioned compression and wearing comfortableness, it is preferable that the garment bodies 104 and 204 to which the electrodes 101a and 101b are attached and the under belt 114 are formed from a woven fabric composed of elastic yarns and inelastic yarns. The woven fabric composed of the elastic yarn and the inelastic yarn is excellent in the stretch property of the clothing fabric, and the above-mentioned compression can be realized.

The material of the elastic yarn to be applied to the woven fabric is not particularly limited, and examples thereof that can be used may include polyurethane elastic fibers, polyether ester elastic fibers, polyamide elastic fibers, polyolefin elastic fibers, so-called rubber yarns in the form of yarn made of natural rubber, synthetic rubber, and semi-synthetic rubber, and special fibers formed by dipping or coating synthetic fibers in or with rubber. Polyurethane elastic fibers are particularly suitable from the viewpoint of durability.

The material of the inelastic yarn applied to the woven fabric is not particularly limited, and examples thereof that can be used may include polyester-based synthetic fibers such as polyethylene terephthalate, polytrimethylene terephthalate, and polybutylene terephthalate, and polyamide-based synthetic fibers such as nylon. As the material of the inelastic yarn, fibers obtained by adding an additive such as titanium oxide to the fiber described above may be used, or fibers modified with a polymer for imparting a function may also be used. In addition, the cross-sectional shape of the filament unit of the inelastic yarn is not specified, and various different cross-sectional yarns typified by a round shape, a triangle shape, an octaleaf shape, a flat shape, and a Y shape may also be used. Further, as the inelastic yarn, a core sheath or a side-by-side type composite yarn made of polymers having different viscosities may also be used. In addition, a false twist processing yarn obtained by subjecting these raw yarns to false twist processing may be used. Further, synthetic fibers such as polyacrylonitrile and polypropylene, regenerated fibers such as rayon, polynosic, and cuprammonium rayon, semisynthetic fibers such as acetate and triacetate, and natural fibers such as cotton, hemp, wool, and silk may be used in accordance with the required characteristics. As described above, an optimum material may be appropriately selected as the inelastic yarn in accordance with the application.

As for the woven or knitted fabric using the elastic yarn and the inelastic yarn, a weaving or knitting method is not particularly limited as long as the compression described above can be achieved. For example, the woven fabric can be produced by a plain weave or twill weave method using a covered yarn in which the elastic yarn is used as a core yarn and the inelastic yarn is used as a sheath yarn to cover the core yarn as a warp yarn and a weft yarn. A circular knit fabric can be produced by a plain knitting or interlock knitting method using the covered yarn. The fabric made by circular knitting can also be produced by bare-yarn plain knitting or bare-yarn interlock knitting in which the inelastic yarn and the elastic yarn are aligned and knitted. Warp knitting may be performed by double denbigh knitting in which the inelastic yarn is used in a front reed and the elastic yarn is used in a back reed, or half knitting. The clothing fabrics made by the circular knitting or the warp knitting are more preferably adopted since they are excellent in the stretchability, cause less disturbance in the compression during the exercise due to smooth stretching and shrinking of the fabrics, and are suitable for the innerwear or the like.

In the crop top type biosignal detecting garment 100 and the brassiere type biosignal detecting garment 200, the electrodes 101a and 101b are disposed in the vicinity of the left and right under busts of the under belt 114, respectively, one of the electrodes 101a and 101b is used as a different electrode, and any of the electrodes 101a and 101b other than the different electrode is used as an indifferent electrode (reference biopotential electrode), and a potential difference between the different electrode and the indifferent electrode is detected as an electrocardiographic waveform. Although FIGS. 1 and 2 exemplify the case where the two electrodes 101a and 101b are used, the number of the electrodes is not limited as long as the number is two or more.

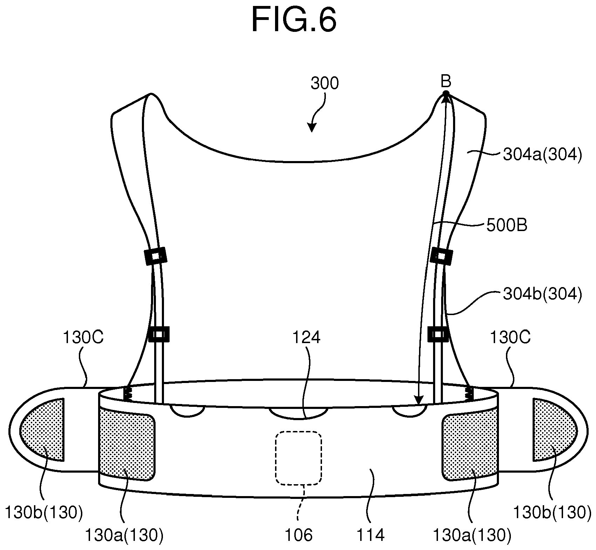

The biosignal detecting garment of the present invention may have a structure as shown in FIG. 6 in addition to the crop top type biosignal detecting garment 100 and the brassiere type biosignal detecting garment 200 shown in FIGS. 1 and 2. FIG. 6 is a schematic diagram of a chest belt type (brassiere type) biosignal detecting garment 300 according to a third embodiment of the present invention. The chest belt type (brassiere type) biosignal detecting garment 300 is a garment including a garment body 304 having straps 304a and a back body piece 304b, and an under belt 114 as parts.

The chest belt type (brassiere type) biosignal detecting garment 300 of the third embodiment includes the garment body 304, the under belt 114 disposed in the lower portion of the garment body 304, at least two electrodes 101a and 101b (not shown) formed of an electroconductive fiber, a connector 102 (not shown) for mounting a measurement device 106 (not shown) that detects biosignals, and wiring portions 103a and 103b (not shown) for electrically connecting the electrodes 101 and the connector 102. As in the first and second embodiments, the electrodes 101a and 101b, the connector 102, and the wiring portions 103a and 103b are installed on the back side of the under belt 114 (side in contact with the body 1). In addition, the same configurations as those of the first and second embodiments can be used for the electrodes 101a and 101b, the connector 102, the wiring portions 103a and 103b, the garment body 304, and the under belt 114.

An opening portion 124 is provided in the front center of the under belt 114. In addition, a hook-and-loop fastener 130a (male) is disposed in the right and left side front portions of the under belt 114, and a handle 130c with a hook-and-loop fastener 130b (female) is attached to the right and left side portions of the under belt 114. The hook-and-loop fasteners 130a, 130b function as fastening tools.

As described above, according to the biosignal detecting garments 100, 200, and 300 of the present invention, it becomes possible to achieve the detection of the biosignals with the form of the garment and thus continuously measure the electrocardiogram or the like for a long period of time without interrupting daily living activities.

Examples

Next, the biosignal detecting garment of the present invention will be described in detail with reference to examples, but the biosignal detecting garment of the present invention is not limited to these examples.

(1) Elongation rate

The elongation rates of the face fabric and the lining fabric were measured in accordance with the elongation rate method D (the fixed load method) in JIS L 1096 (2010) "Testing methods for woven and knitted fabrics".

First, five test pieces each having a width of 50 mm and a length of 300 mm were collected both in the wale direction and in the course direction. The measurement was performed using a tensile tester with a grip interval of 200 mm. After removing sagging and tension, the test piece was fixed to grips while applying an initial load of 29 mN (3 g). The sample was stretched at a tensile speed of 200 mm/min up to 4.9 N (500 g) and then retained for one minute while applying a predetermined load to the sample. After one minute of retention, the grip interval was measured. The elongation rate $E_p$(%) was obtained using the following equation and represented as an average value of five test pieces both in the wale direction and in the course direction.

$$\text{Elongation rate } E_p \ (\%) \ = \ \{(L_1 \ - \ L)/L\} \ \times \ 100$$

L: initial length (mm) of grip interval
$L_1$: length (mm) between marks after being allowed to stand for one minute

(2) Elongation recovery rate

Further, the elongation recovery rate was measured in accordance with the method E (the fixed load method) in JIS L 1096 (2010) "Testing methods for woven and knitted fabrics".
First, five test pieces each having a width of 50 mm and a length of 300 mm were collected both in the wale direction and in the course direction. The measurement was performed using the tensile tester with a grip interval of 200 mm. After removing sagging and tension, the test piece was fixed to grips while applying an initial load of 29 mN (3 g). The sample was stretched at a tensile speed of 200 mm/min up to 4.9 N (500 g) and then retained for one minute while applying a predetermined load to the sample. After one minute of retention, the load was immediately removed and the test piece was further allowed to stand for three minutes. After repeating this operation, the length (mm) of the grip interval was measured again with the initial load.
The elongation recovery rate $E_e$(%) was obtained using the following equation and represented as an average value of five test pieces both in the wale direction and in the course direction.

$$\text{Elongation recovery rate } E_e \ (\%) \ =\{(L_0 \ - \ L_1)/(L_0 \ - \ L)\} \ \times \ 100$$

L: initial length (mm) between marks
$L_0$: length (mm) between marks after being allowed to stand for one minute
$L_1$: length (mm) of grip interval after load being removed and sample being allowed to stand for three minutes and applied with initial load

(3) Under belt width

The width of the under belts of the garments obtained in Examples and Comparative examples was measured.

(4) Length from neck point or apex of strap to upper portion of under belt

As shown in FIG. 5 and FIG. 6, the length from the neck point A to the upper portion of the under belt 114 (the length 500A of a perpendicular line lowered to the under belt 114) or the length from the apex B of the strap to the upper portion of the under belt 114 (the length 500B of a perpendicular line lowered to the under belt 114) in the biosignal detecting garments obtained in Examples and Comparative examples was measured. The measurement was performed on a flat table without applying any tension to the garment or the clothing fabric.

(5) Garment pressure

Air-pack sensors of a contact surface pressure measurement device manufactured by AMI Techno Co., Ltd. were attached to a wearer at a total of two sites, the armpit and the right fifth rib vicinity (the position of the under belt). The garment pressure of the biosignal detecting garment worn over the sensors was measured in a standing state kept for 10 seconds and a bent-forward state kept for 10 seconds, and average values of three garment pressure measurement results were obtained.

(6) Data collectability, wearing comfortableness, and ease of putting-on/taking-off

Five testers were requested to wear the biosignal detecting garments obtained in Examples and Comparative examples, and data collectability of the biosignals (biosignal detection accuracy), wearing comfortableness, and ease of putting-on/taking-off were evaluated into five grades. An average value in each evaluation was obtained. Evaluation standards are shown in Table 1.

The data collectability of the biosignals is an evaluation item that determines the presence/absence of noise in the biosignal (an electrocardiographic waveform) obtained from the biosignal detecting garments in Examples and Comparative examples when the wearer was in a resting state for 10 seconds and then performed arm swinging 10 times, waist twisting to right and left 10 times, and forward bending 10 times. If the noise was not introduced into the biosignal (the electrocardiographic waveform), the garment was determined as having the excellent data collectability, while if the noise was introduced into the biosignal, the garment was determined as having the poor data collectability.

Table 1

| Evaluation | Data Collectability of Biosignal | Wearing Comfortableness | Ease of Putting-on/Taking-off |
|---|---|---|---|
| 5 | Capable of Collecting | Excellent | Very Easy to Take Off |
| 4 | | Good | Easy to Take Off |
| 3 | | Average | Average |
| 2 | | Below Average | Slightly Hard to Take Off |
| 1 | Incapable of Collecting | Poor | Hard to Take Off |

Example 1

In Example 1, the crop top type biosignal detecting garment 100 shown in FIG. 1 was produced by sewing. As a main fabric of the garment body 104 and the under belt 114, a polyester fiber of 56T-22F formed of polyethylene terephthalate and a polyurethane fiber were used to produce a circular knit fabric. Further, the electrodes 101 were produced by applying a dispersion liquid prepared by dispersing 1.0 wt% PEDOT/PSS as an electroconductive polymer and 5.0 wt% acryl-based thermosetting resin as a binder in a mixed solvent of water and ethanol (44 wt% water, 50 wt% ethanol) to the above-mentioned circular knit fabric in the agent application amount of 15 g/m$^2$ using a known gravure coating method. Further, the biosignal detecting garment 100 of the following specifications was produced by using the wiring portions 103, each of which was woven in a belt shape having a width of 1.5 cm and a length of 5.0 cm using a yarn in which a nylon fiber was covered with a silver thread, and the waterproof electrically insulating films provided with a hot-melt adhesive serving as the electrically insulating members 105. Note that, as the connector 102, a metal snap fastener for clothing manufactured by YKK Inc. was used.

In the garment body 104, the length (500A) from the neck point A of the front body piece 104a to the upper portion of the under belt 114 was set to 24 cm and two under belts 114 each having a length of 65 cm and a width of 8 cm were sewn to the lower sides of the front body piece 104a and the right and left back body piece 104b to form the garment in a bag shape. In the sewing portions between the front center of the front body piece 104a and the under belts 114, one of the under belts 114 was not sewn to the front body piece 104a to provide an opening portion 124. Further, the fastening tools 130 were installed on right and left sides of the back center of the under belt 114. The electrodes 101a and 101b, the wiring portions 103a and 103b, and the connector 102 were adhered to the electrically insulating member 105 that was adhered to the back surface side (the side in contact with the skin) of the under belt 114, and the wiring portions 103a and 103b were further covered with the electrically insulating member 105. The electrodes 101a and 101b were disposed in positions 5.0 cm away from the front center of the under belt 114 to the right and left. The measurement device 106 serving as the electrocardiograph measurement device was connected to the connector 102 having the electrode 101b disposed on the left side as a positive different electrode (anode) and the electrode 101a disposed on the right side as a negative different electrode (cathode) to acquire the electrocardiographic waveform. This allowed the measurement device 106 to detect the electrocardiographic waveform.

Example 2

A biosignal detecting garment was produced by the same manner and procedure as that of Example 1 except for its pattern. In Example 2, the brassiere type biosignal detecting garment 200 having the garment shape shown in FIG. 2 was produced by sewing. The length (500B) from the apex B of the strap to the upper portion of the under belt 114 was set to 24 cm and two under belts 114 each having a length of 65 cm and a width of 8 cm were sewn to the lower side of the front body piece 204a to form the garment having a bag shape. Right and left shoulder portions of the front body piece 204a were connected to right and left back side portions of the under belt 114 using straps 204b. In the sewing portions between the front center of the front body piece 204a and the under belts 114, one of the under belts 114 was not sewn to provide an opening portion 124. Further, the fastening tools 130 were installed on right and left sides of the back center of the under belt 114. The electrodes 101a and 101b, the wiring portions 103a and 103b, and the connector 102 were adhered to the electrically insulating member 105 that was adhered to the back surface side (the side in contact with the skin) of the under belt 114, and the wiring portions 103a and 103b were further covered with the electrically insulating member 105. The electrodes 101a and 101b were disposed in positions 5.0 cm away from the front center of the under belt 114 to the right and left. The measurement device 106 serving as the electrocardiograph measurement device was connected to the connector 102 having the electrode 101b disposed on the left side as a positive different electrode (anode) and the electrode 101a disposed on the right side as a negative different electrode (cathode) to acquire the electrocardiographic waveform. This allowed the measurement device 106 to detect the electrocardiographic waveform.

Example 3

In Example 3, the chest belt-type (brassiere type) biosignal detecting garment 300 with shoulder straps shown in FIG. 6 was produced by sewing. As a main fabric of the under belt 114 and a garment body 304, a polyester fiber of 56T-22F formed of polyethylene terephthalate and a polyurethane fiber were used to produce a circular knit fabric. Further, the electrodes 101 were produced by applying a dispersion liquid prepared by dispersing 1.0 wt% PEDOT/PSS as an electroconductive polymer and 5.0 wt% acryl-based thermosetting resin as a binder in a mixed solvent of water and ethanol (44 wt% water, 50 wt% ethanol) to the above-mentioned circular knit fabric in the agent application amount of 15 g/m$^2$ using a known gravure coating method. Further, the biosignal detecting garment 300 of the following specifications was produced by using the wiring portions 103, each of which was woven in a belt shape having a width of 1.5 cm and a length of 5.0 cm using a yarn in which a nylon fiber was covered with a silver thread, and the waterproof electrically insulating films provided with the hot-melt adhesive as the electrically insulating members 105 (not illustrated). Note that, as the connector, the metal snap fastener for clothing manufactured by YKK Inc. was used.

In the biosignal detecting garment 300 produced in Example 3, the length (500B) from the apex B of the strap 304a to the upper portion of the under belt 114 was set to 24 cm. Two under belts 114 each having a length of 65 cm and a width of 8 cm were sewn to form the garment in a bag shape. The front center of the under belt 114 was not sewn to provide the opening portion 124. Further, hook-and-loop fasteners 130a (male surfaces) were sewn to right and left side front portions of the under belt 114 and handles 130c with hook-and-loop fasteners 130b (female surfaces) attached thereto were inserted in right and left side portions of the under belts 114, allowing adjustment of the chest circumference size. The electrodes 101, the connector 102, the wiring portions 103, and the electrically insulating members 105 were disposed similarly to those in Examples 1 and 2. The measurement device 106 serving as the electrocardiograph measurement device was connected to the connector 102 having the electrode 101b disposed on the left side as a positive different electrode (anode) and the electrode 101a disposed on the right side as a negative different electrode (cathode) to acquire the electrocardiographic waveform. This allowed the measurement device 106 to detect the electrocardiographic waveform.

Comparative example 1

A flat rubber having a width of 6.0 cm was formed into a belt having a length of 65 cm using a yarn in which a polyurethane fiber is covered with a nylon fiber. The electrodes 101a and 101b, the connector 102, and the wiring portions 103a and 103b produced similarly to those in Example 1 were installed in the belt thus produced to produce a biosignal detection belt.

Comparative example 2

A biosignal detecting garment was produced similarly to that in Example 1 except that the electrodes 101a and 101b were disposed in right and left chest lower portions of the front body piece 104a instead of in the under belt 114.

Comparative example 3

A biosignal detecting garment was produced similarly to that in Example 1 except that the patterns of the front body piece 104a and the right and left back body piece 104b were changed and the length (500A) from the neck point A of the front body piece 104a to the upper portion of the under belt 114 was set to 18 cm.

Comparative example 4

A biosignal detecting garment was produced similarly to that in Example 1 except that the patterns of the front body piece 104a and the right and left back body piece 104b were changed and the length (500A) from the neck point A of the front body piece 104a to the upper portion of the under belt 114 was set to 40 cm.

Comparative example 5

A biosignal detecting garment was produced similarly to that in Example 1 except that the pattern and configuration of the garment was changed to those of a T-shirt and the electrodes 101a and 101b were disposed in right and left lower epigastric portions.

The garment shape, the mixing ratio, the elongation rate (%) of the wale and the course, and the elongation recovery rate (%) of the wale and the course in Examples 1 to 3 and Comparative examples 1 to 5 were summarized in Table 2.

Table 2

|  | Garment Shape | Mixing Ratio | Elongation Rate (%) | | Elongation Recovery Rate (%) | |
|---|---|---|---|---|---|---|
|  |  |  | Wale | Course | Wale | Course |
| Example 1 | Crop Top Type | PET:92 PU: 8 | 91 | 41.5 | 92 | 92.2 |
| Example 2 | Brassiere Type | PET:92 PU: 8 | 91 | 41.5 | 92 | 92.2 |
| Example 3 | Brassiere Type | PET:92 PU: 8 | 91 | 41.5 | 92 | 92.2 |
| Comparative Example 1 | Belt | Flat Rubber : 100 | 36 | 20.0 | 95 | 96.0 |
| Comparative Example 2 | Crop Top Type | PET:92 PU: 8 | 91 | 41.5 | 92 | 92.2 |
| Comparative Example 3 | Crop Top Type | PET:92 PU: 8 | 91 | 41.5 | 92 | 92.2 |
| Comparative Example 4 | Crop Top Type | PET:92 PU: 8 | 91 | 41.5 | 92 | 92.2 |
| Comparative Example 5 | T-shirt Type | PET:92 PU: 8 | 91 | 41.5 | 92 | 92.2 |

The under belt width (cm), the height (cm) from the neck point A or the apex B of the strap to the under belt, the garment pressure (armpit) (kPa) in the standing and forward bending states, and the garment pressure (fifth rib vicinity) (kPa) in the standing and forward bending states in Examples 1 to 3 and Comparative examples 1 to 5 were summarized in Table 3.

Table 3

|  | Under Belt Width (cm) | Length from Neck Point or Apex of Strap to Upper Portion of Under Belt (cm) | Garment Pressure (Armpit) (kPa) | | Garment Pressure (Fifth rib vicinity) (kPa) | |
|---|---|---|---|---|---|---|
|  |  |  | Standing | Bent-forward | Standing | Bent-forward |
| Example 1 | 8.5 | 24.0 | 1.3 | 1.65 | 0.7 | 0.8 |
| Example 2 | 8.5 | 24.0 | 1.3 | 1.65 | 0.7 | 0.8 |

(continued)

|  | Under Belt Width (cm) | Length from Neck Point or Apex of Strap to Upper Portion of Under Belt (cm) | Garment Pressure (Armpit) (kPa) | | Garment Pressure (Fifth rib vicinity) (kPa) | |
|---|---|---|---|---|---|---|
|  |  |  | Standing | Bent-forward | Standing | Bent-forward |
| Example 3 | 8.5 | 24.0 | 2.2 | 2.4 | 2.2 | 2.3 |
| Comparative Example 1 | 6.0 | - | - | - | 2.6 | 3.0 |
| Comparative Example 2 | 8.5 | 24.0 | 1.3 | 1.65 | 0.7 | 0.8 |
| Comparative Example 3 | 8.5 | 18.0 | 1.3 | 1.65 | 0.7 | 0.8 |
| Comparative Example 4 | 8.5 | 40.0 | 1.3 | 1.65 | 0.7 | 0.8 |
| Comparative Example 5 | - | - | 0.2 | 0.40 | - | - |

The data collectability of the biosignal (the biosignal detection accuracy), the wearing comfortableness, and the ease of putting-on/taking-off in Examples 1 to 3 and Comparative examples 1 to 5 were summarized in Table 4.

Table 4

|  | Data Collectability of Biosignal | Wearing Comfortableness | Ease of Putting-on/Taking-off |
|---|---|---|---|
| Example 1 | 5 | 5 | 5 |
| Example 2 | 5 | 5 | 5 |
| Example 3 | 5 | 5 | 5 |
| Comparative Example 1 | 5 | 2 | 5 |
| Comparative Example 2 | 1 | 5 | 5 |
| Comparative Example 3 | 1 | 5 | 5 |
| Comparative Example 4 | 1 | 5 | 5 |
| Comparative Example 5 | 5 | 5 | 2 |

As is evident from Table 4, it was found that Examples 1 to 3 within the range of the biosignal detecting garments of the present invention were excellent in the biosignal detection accuracy and the wearing comfortableness, while in Comparative examples 1 to 5 outside the range of the biosignal detecting garments of the present invention, Comparative example 1 was inferior to the present invention in the wearing comfortableness, Comparative examples 2 to 4 were inferior to the present invention in the biosignal detection accuracy, and Comparative example 5 was inferior to the present invention in the ease of putting-on/taking-off.

Reference Signs List

1 body
3 brassiere
100 crop top type biosignal detecting garment
101a, 101b electrode
102 connector
103a, 103b wiring portion
104, 204, 304 garment body

105 electrically insulating member
106 measuring device
114 under belt
124 opening portion
130 fastening tool
200 brassiere type biosignal detecting garment
300 chest belt type (brassiere type) biosignal detecting garment
500A length from neck point A to upper portion of under belt
500B length from apex B of strap to upper portion of under belt
A neck point
B apex of strap


**Claims**

1.  A biosignal detecting garment comprising:

    a crop top type or brassiere type garment body (104, 204, 304);
    an under belt (114) that have a fastening tool (130) capable of adjusting a chest circumference size and that is disposed in a lower portion of the garment body(104, 204, 304) ;
    at least two electrodes (101) formed of an electroconductive fiber;
    a connector (102) for installing a measurement device configured to detect a biosignal; and
    a wiring portion (103) configured to electrically connect the electrodes (101) and the connector (102), wherein the electrodes (101), the connector (102), and the wiring portion (103) are installed in the under belt (114),
    the garment body (104, 204, 304) has a length (500B) from an apex of a shoulder portion of the garment body (104, 204, 304) to an upper portion of the under belt (114) of 20.0 cm or more and 35.0 cm or less,
    the under belt (114) is formed in a bag shape with two pieces stacked, and
    an opening portion (124) with a range of 1.0 cm or more and 10.0 cm or less is provided at a center of a front body piece.

2.  The biosignal detecting garment according to claim 1, wherein a width of the under belt (114) is 6.0 cm or more.

3.  The biosignal detecting garment according to claim 1 or 2, wherein
    the garment body (104, 204, 304) and the under belt (114) are formed of a stretchable clothing fabric, an elongation rate of the clothing fabric in a longitudinal or lateral direction is 30.0% or more and 100.00% or less, and an elongation recovery rate of the clothing fabric is 80.0 % or more and 100.0% or less.

4.  The biosignal detecting garment according to any one of claims 1 to 3, wherein a garment pressure of the garment body (104, 204, 304) is 1.0 kPa or less, and a garment pressure of the under belt (114) is 0.2 kPa or more and 2.5 kPa or less.


**Patentansprüche**

1.  Biosignaldetektionskleidungsstück, das Folgendes umfasst:

    einen Kleidungsstückkörper (104, 204, 304) vom Bustiertyp oder Büstenhaltertyp;
    einen Untergurt (114), der ein Befestigungselement (130) aufweist, das in der Lage ist, eine Brustumfangsgröße anzupassen, und der in einem unteren Abschnitt des Kleidungsstückkörpers (104, 204, 304) angeordnet ist;
    zumindest zwei Elektroden (101), die aus einer elektrisch leitenden Faser ausgebildet sind;
    einen Steckverbinder (102) zum Anbringen einer Messvorrichtung, die dazu ausgelegt ist, ein Biosignal zu detektieren; und
    einen Verdrahtungsabschnitt (103), der dazu ausgelegt ist, die Elektroden (101) und den Steckverbinder (102) elektrisch miteinander zu verbinden,
    wobei die Elektroden (101), der Steckverbinder (102) und der Verdrahtungsabschnitt (103) im Untergurt (114) angebracht sind,
    wobei der Kleidungsstückkörper (104, 204, 304) eine Länge (500B) von einem Scheitelpunkt eines Schulterabschnitts des Kleidungsstückkörpers (104, 204, 304) bis zu einem oberen Abschnitt des Unterguts (114) von 20,0 cm oder mehr und 35,0 cm oder weniger aufweist,
wobei der Untergurt (114) in einer Beutelform ausgebildet ist, bei der zwei Stücke aufeinandergestapelt sind, und
wobei ein Öffnungsabschnitt (124) mit einem Bereich von 1,0 cm oder mehr und 10,0 cm oder weniger in der Mitte eines vorderen Körperstücks bereitgestellt ist.

2. Biosignaldetektionskleidungsstück nach Anspruch 1, wobei die Breite des Untergurts (114) 6,0 cm oder mehr beträgt.

3. Biosignaldetektionskleidungsstück nach Anspruch 1 oder 2, wobei der Kleidungsstückkörper (104, 204, 304) und der Untergurt (114) aus einem dehnbaren Bekleidungsstoff ausgebildet sind, wobei die Dehnungsrate des Bekleidungsstoffs in Längs- oder Seitenrichtung 30,0 % oder mehr und 100,0 % oder weniger beträgt und die Dehnungserholungsrate des Bekleidungsstoffs 80,0 % oder mehr und 100,0 % oder weniger beträgt.

4. Biosignaldetektionskleidungsstück nach einem der Ansprüche 1 bis 3, wobei der Kleidungsstückdruck des Kleidungsstückkörpers (104, 204, 304) 1,0 kPa oder weniger beträgt und der Kleidungsstückdruck des Untergurts (114) 0,2 kPa oder mehr und 2,5 kPa oder weniger beträgt.

**Revendications**

1. Vêtement de détection de biosignal comprenant :

un corps de vêtement de type brassière ou de type soutien-gorge (104, 204, 304) ;
une ceinture inférieure (114) qui présente un outil de fixation (130) capable d'ajuster une taille de tour de poitrine et qui est disposé dans une partie inférieure du corps de vêtement (104, 204, 304) ;
au moins deux électrodes (101) formées d'une fibre électroconductrice ;
un connecteur (102) pour installer un dispositif de mesure configuré pour détecter un biosignal ; et
une partie de câblage (103) configurée pour connecter électriquement les électrodes (101) et le connecteur (102), dans lequel
les électrodes (101), le connecteur (102) et la partie de câblage (103) sont installés dans la ceinture inférieure (114),
le corps de vêtement (104, 204, 304) présente une longueur (500b) à partir d'un sommet d'une partie d'épaule du corps de vêtement (104, 204, 304) vers une partie supérieure de la ceinture inférieure (114) de 20,0 cm ou plus et de 35,0 cm ou moins,
la ceinture inférieure (114) est formée en forme de poche avec deux pièces empilées, et
une partie d'ouverture (124) avec une plage de 1,0 cm ou plus et de 10,0 cm ou moins est prévue au centre d'une pièce de corps avant.

2. Vêtement de détection de biosignal selon la revendication 1, dans lequel une largeur de la ceinture inférieure (114) est de 6,0 cm ou plus.

3. Vêtement de détection de biosignal selon la revendication 1 ou 2, dans lequel
le corps de vêtement (104, 204, 304) et la ceinture inférieure (114) sont formés d'un tissu de confection extensible, un taux d'allongement du tissu de confection dans une direction longitudinale ou latérale est de 30,0 % ou plus et de 100,00 % ou moins, et un taux de récupération d'allongement du tissu de confection est de 80,0 % ou plus et de 100,0 % ou moins.

4. Vêtement de détection de biosignal selon l'une quelconque des revendications 1 à 3, dans lequel une pression de vêtement du corps de vêtement (104, 204, 304) est de 1,0 kPa ou moins, et une pression de vêtement de la ceinture inférieure (114) est de 0,2 kPa ou plus et de 2,5 kPa ou moins.

# FIG.1

(a)

(b)

(c)

# FIG.2

(a)

204a(204)

200

124

106

r1

114

(b)

204a(204)

200

(103)
103a    102

106

(103)
103b

X    X'

101a(101)

114

r2

101b(101)

105

105    102

102

(c)

204b(204)    204b(204)

200

204a(204)

130    130    114

# FIG.3

SKIN SIDE

101a(101)

105

105

105

103a(103)

102

114

FRONT SIDE

# FIG.4

# FIG.5

(a)

100

A

500A

104a(104)

114

106

124

(b)

200

B

500B

204a(204)

114

106

124

# FIG.6

**EP 3 510 922 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015100673 A
- JP 2012188799 A
- US 8224418 B
- JP 2008503287 W
- JP 6070896 A
- JP 1552253 A
- US 20140343390 A
- US 20140009731 A
- JP 2013185283 A